(19) Europäisches Patentamt
European Patent Office
Office européen des brevets

(11) Veröffentlichungsnummer: **0 437 820 A2**

# (12) EUROPÄISCHE PATENTANMELDUNG

(21) Anmeldenummer: **90125272.6**

(51) Int. Cl.5: **C07H 15/252**, C07H 23/00

(22) Anmeldetag: **21.12.90**

(30) Priorität: **27.12.89 DE 3943029**

(43) Veröffentlichungstag der Anmeldung: **24.07.91 Patentblatt 91/30**

(84) Benannte Vertragsstaaten: **AT BE CH DE DK ES FR GB GR IT LI LU NL SE**

(71) Anmelder: **BEHRINGWERKE Aktiengesellschaft
Postfach 1140
W-3550 Marburg 1(DE)**

(72) Erfinder: **Kolar, Cenek, Dr.
Deutschhausstrasse 20
W-3550 Marburg(DE)**

(74) Vertreter: **Klein, Otto, Dr. et al
Hoechst AG Zentrale Patentabteilung
Postfach 80 03 20
W-6230 Frankfurt am Main 80(DE)**

## (54) Verfahren zur Herstellung von Glycosyl-Anthracyclinonen.

(57) Es wird ein Verfahren zur Herstellung von 7-O-Glycosylanthracyclinonen, die der nachfolgenden allgemeinen Formel I entsprechen, beschrieben, worin die Reste

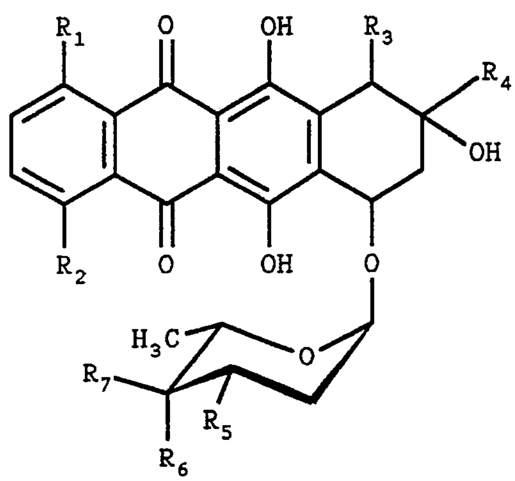

I

| | |
|---|---|
| $R^1$ | H oder OH |
| $R^2$ | H, OH oder $OCH_3$ |
| $R^3$ | H, $COOCH_3$, OH oder O-Acyl-Schutzgruppe |
| $R^4$ | $CH_2CH_3$, $COCH_3$, $COCH_2OH$ oder $COCH_2O$-Acyl-Schutzgruppe |
| $R^5$ | $NH_2$, NH-Acyl-Schutzgruppe, OH oder O-Acyl-Schutzgruppe |
| $R^6$ | H, OH, O-Acyl-Schutzgruppe, $NH_2$ oder NH-Acyl-Schutzgruppe |
| $R^7$ | H, OH oder O-Acyl-Schutzgruppe |

bedeuten, wobei eine Acyl-Schutzgruppe eine Acetyl-, Mono-, Di- oder Trihalogenacetylgruppe mit Halogen, Fluor oder Chlor oder eine Benzoyl- oder p-Nitrobenzoylgruppe darstellt, ausgehend von einer Anthracyclinon-Verbindung der Formel II

EP 0 437 820 A2

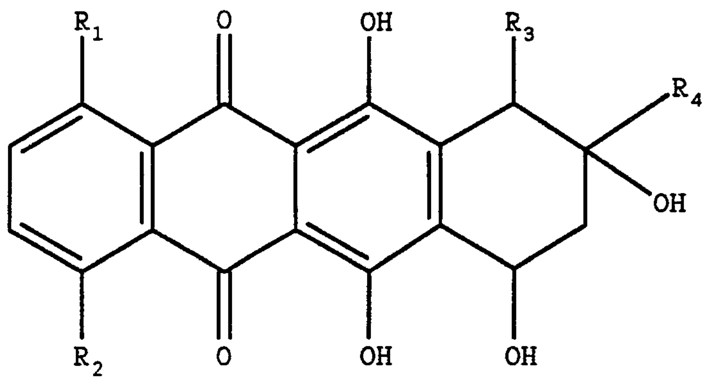

II

worin

$R^1$ H oder OH
$R^2$ H, OH oder $OCH_3$
$R^3$ H, $COOCH_3$ oder O-Acyl-Schutzgruppe und
$R^4$ $CH_2CH_3$, $COCH_3$, $COCH_2$-O-Acyl-Schutzgruppe

bedeuten, und einen funktionalisierten Kohlenhydrat-Baustein der Formel III

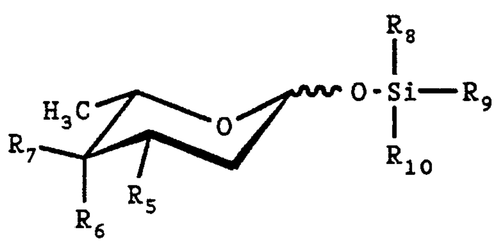

III

worin

$R^5$ NH-Acyl-Schutzgruppe oder O-Acyl-Schutzgruppe
$R^6$ H, NH-Acyl-Schutzgruppe oder O-Acyl-Schutzgruppe
$R^7$ H, O-Acyl-Schutzgruppe und
$R^8$, $R^9$ und $R^{10}$ $(C_1-C_4)$-Alkyl

bedeuten, wobei eine Acyl-Schutzgruppe für Aminogruppen bevorzugt eine Trifluoroacetylgruppe und für Hydroxygruppen bevorzugt eine Acetyl-, Trifluoroacetyl-, Chloroacetyl oder p-Nitrobenzoyl-Gruppe sind, in Gegenwart eines Promotors wie Trifluoromethansulfonsäure tri-(C1-C4)-alkylsilyl-esters oder -anhydrides oder $BF_3$-Ethers, in einem organischen wasserfreien Lösungsmittel, gegebenenfalls in Präsenz einer Base oder eines Säurefängers und eines Trockenmittels bei -50° C bis 25° C, wobei eine Verbindung der Formel I, worin die Reste $R^1$, $R^2$, $R^3$, $R^4$, $R^5$, $R^6$ und $R^7$ die oben definierte Bedeutung beibehalten und diese Verbindungen noch einen Deacylierungsschritt mittels einer Alkalilauge oder eines Alkoholates zur Herstellung von zytostatisch wirksamen Verbindungen der Formel I,

worin

$R^1$ H oder OH
$R^2$ H, OH oder $OCH_3$
$R^3$ H, $COOCH_3$ oder OH
$R^4$ $CH_2CH_3$, $COCH_3$ oder $COCH_2OH$
$R^5$ $NH_2$ oder OH
$R^6$ H, OH oder $NH_2$ und
$R^7$ H oder OH

bedeuten, entsteht.

# VERFAHREN ZUR HERSTELLUNG VON GLYCOSYL-ANTHRACYCLINONEN

Die vorliegende Erfindung bezieht sich auf ein Verfahren, besonders auf ein Glycosylierungsverfahren, zur Herstellung von 7-O-Glycosyl-anthracyclinonen, die aufgrund ihrer zytostatischen Wirksamkeit für die Behandlung von Krebserkrankungen geeignet sind.

Die Substanzklasse der Anthracycline ist in der Fachliteratur eingehend beschrieben. Doxorubicin und sein 14-Desoxy-Analogon Daunorubicin sind hier als die erfolgreichsten Vertreter dieser Substanzklasse genannt, die in der Klinik zur Behandlung einer großen Anzahl von festen Tumoren und Leukämien eingesetzt werden. Weitere Analoga, die sowohl in dem Aglyconteil als auch in der Kohlenhydrateinheit modifiziert wurden, sind kürzlich in die Klinik eingeführt worden oder befinden sich in der klinischen Prüfung.

Die chemische Herstellung von Anthracyclinen wird ausgehend von einem funktionalisierten Kohlenhydratbaustein als Donor und einem Anthracyclinon als Akzeptor in der Gegenwart eines Promotors durchgeführt. Hierbei wird die Hydroxygruppe am C-7 Atom des Anthracyclinon-Akzeptors mit einem Kohlenhydratbaustein unter Herausbildung einer α-O-glycosidischen Verknüpfung glycosyliert.

Zur Glycosylierung von Anthracyclinonen wurden folgende funktionalisierte Kohlenhydratbausteine verwendet:

1) Glycosylhalogenide unter Verwendung von Silbersalzen als Promotor (F. Arcamone in "Doxorubicin", Academic Press, 1981, S. 82-92 und S. 194-258.

2) Glycosylhalogenide unter Verwendung von Quecksilbersalzen als Promotor (T.M. Smith et al., J. Org. Chem. 42, 3653 (1977), F. Arcamone et al., Cancer Treat Rep. 60, 829 (1976).

3) Glycale unter Verwendung von Säure als Promotor (H. Umezawa et al., J. Antibiotics 33, 1581 (1980)).

4) Glycale unter Verwendung von N-Jodsuccinimid (D. Horton et al. in "Anthracycline Antibiotics", Editor H. S. El Khadem, Academic Press, 1982, S. 197 - 224).

5) 1-0-Acyl-Kohlenhydrat-Donors unter Verwendung von Trimethylsilyltriflat als Promotor (EP-0 143 323/ 1988).

Nach der Methode 1 und 2 sind α-Glycoside selektiv herstellbar, da jedoch die Glycosylhalogenide sehr instabil sind, ist ihre Verwendung in der Glycosidsynthese, vor allem im technischen Maßstab, sehr problematisch. Darüber hinaus werden hierbei teure Silbersalze bzw. giftige Quecksilbersalze verwendet.

Nach der Methode 3 sind α-Glycoside nur als Gemisch der α - und ß-Produkte herstellbar. Nach der Methode 4 sind nur 2'-Halogen-Anthracycline herstellbar.

Nach der Methode 5 sind α-Glycoside selektiv herstellbar, da jedoch das Glycosyldonor als ein Gemisch der α- und ß-O-Acyl-Derivate vorliegt, wobei bei der Glycosylierung bevorzugt die ß-O-Acyl-Derivate abreagieren, verbleibt meist die α-O-Acyl-Komponente während der Reaktion unumgesetzt. Aufwendige Chromatographie ist bei der Reinigung der gewünschten α-Glycoside notwendig.

Überraschenderweise hat es sich bei der Glycosylierung von 1 Äquivalent (äq.) ε-Rhodomycinon mit einem äq. 1-O-tert.-Butyldimethylsilyl-daunosamin-Derivat als Donor ergeben, daß selektiv α-Daunosaminylrhodomycinone in hoher Ausbeute entstehen. Bei dieser Synthese der α-Glycosyl-anthracyclinone wurden früher 3 äq. Glycosylierungskomponenten (4-O-p-Nitro-benzoyl-3-N-tri-fluoroacetyl-α-L-daunosaminyl-chlorid) benötigt.

Aufbauend auf diesen Erkenntnissen hat es sich die vorliegende Erfindung zur Aufgabe gestellt, ein neues Verfahren zur Glycosylierung von Anthracyclinonen mit 1-O-Trialkylsilyl-Zuckern zu entwickeln.

Diese Aufgabe wird erfindungsgemäß durch das Verfahren zur Herstellung eines Anthracyclins der Formel I gelöst,

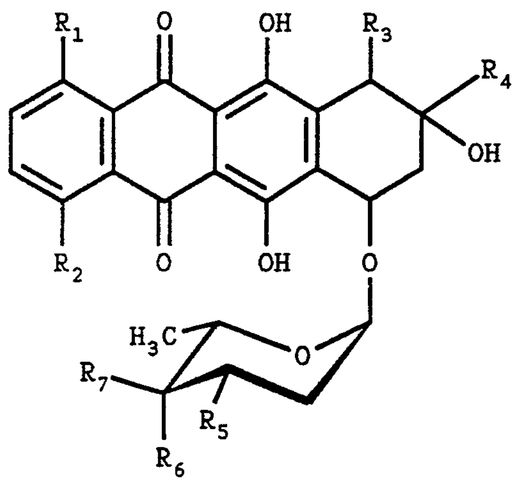

I

worin
$R^1$ H oder OH
$R^2$ H, OH oder $OCH_3$
$R^3$ H, $COOCH_3$, OH oder O-Acyl-Schutzgruppe
$R^4$ $CH_2CH_3$, $COCH_3$, $COCH_2OH$ oder $CO\text{-}CH_2O$-Acyl-Schutzgruppe
$R^5$ $NH_2$,NH-Acyl-Schutzgruppe, OH oder O-Acyl-Schutzgruppe
$R^6$ H, OH, O-Acyl-Schutzgruppe, $NH_2$ oder NH-Acyl-Schutzgruppe
$R^7$ H, OH oder O-Acyl-Schutzgruppe
bedeuten. Unter einer Acyl-Schutzgruppe ist eine in der Kohlenhydratchemie übliche Acylgruppe zu verstehen, die sich von einer $C_1$-$C_4$-Alkansäure, Mono-, Di- oder Trihalogenessigsäure sowie Benzoesäure ableitet.

Vorzugsweise ist eine Acyl-Schutzgruppe Acetyl-, Mono-, Di- oder Trihalogenacetyl mit Halogen, Fluor oder Chlor oder Benzoyl oder p-Nitrobenzoyl.

Bevorzugt sind Verbindungen der Formel I, worin $R^1$, $R^2$, $R^3$, $R^4$, $R^5$, $R^6$ und $R^7$ ihre oben definierte Bedeutung beibehalten, wobei eine Acyl-Schutzgruppe Acetyl, Chloroacetyl, Trifluoroacetyl oder p-Nitrobenzoyl darstellt.

Die Verbindungen der Formel I können gegebenenfalls als Ammonium-Salze vorliegen.

Das erfindungsgemäße Verfahren zur Herstellung einer Verbindungen der Formel I ist dadurch gekennzeichnet, daß man eine Anthracyclinon-Verbindung der Formel II

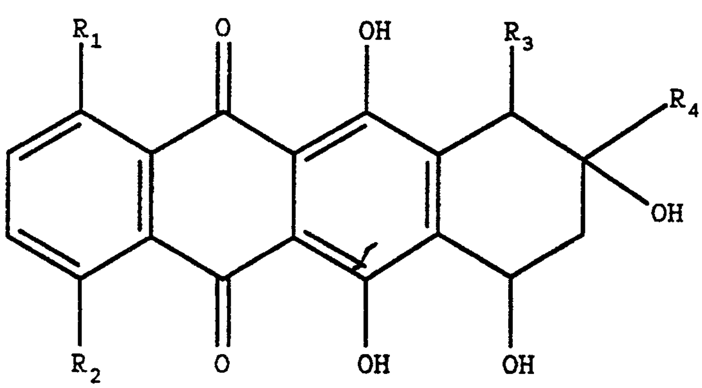

II

worin
$R^1$ H oder OH
$R^2$ H, OH oder $OCH_3$
$R^3$ H, $COOCH_3$ oder O-Acyl-Schutzgruppe und
$R^4$ $CH_2CH_3$, $COCH_3$, $CO\text{-}CH_2$-O-Acyl-Schutzgruppe
bedeuten,
mit einem funktionalisierten Kohlenhydrat-Baustein der Formel III

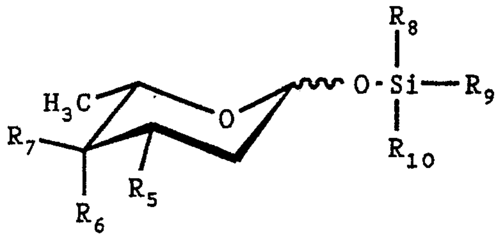

III

worin

$R^5$ NH-Acyl-Schutzgruppe oder O-Acyl-Schutzgruppe

$R^6$ H, NH-Acyl-Schutzgruppe oder O-Acyl-Schutzgruppe

$R^7$ H oder O-Acyl-Schutzgruppe und

$R^8$ , $R^9$ und $R^{10}$ ($C_1$-$C_4$)-Alkyl

bedeuten, wobei eine Acyl-Schutzgruppe für Aminogruppen bevorzugt Trifluoroacetyl und für Hydroxygruppen bevorzugt Acetyl, Trifluoroacetyl, Chloroacetyl oder p-Nitrobenzoyl sind, in Gegenwart eines Promotors wie ein Trifluoromethansulfonsäure-tri-($C_1$-$C_4$)-alkylsilyl-ester oder -anhydrid oder $BF_3$x Ether, in einem organischen wasserfreien Lösungsmittel, gegebenenfalls in der Präsenz einer Base oder eines Säurefängers und eines Trockenmittels bei -50° C bis 25° C zu einer Verbindung der Formel I umsetzt, worin die Reste $R^1$, $R^2$, $R^3$, $R^4$, $R^5$, $R^6$ und $R^7$ die oben definierte Bedeutung beibehalten und diese Verbindungen gegebenenfalls vorzugsweise mittels einer Alkalilauge oder eines Alkoholates zu zytostatisch wirksamen Verbindungen der Formel I,

worin

$R^1$ H oder OH

$R^2$ H, OH oder $OCH_3$

$R^3$ H, $COOCH_3$ oder OH

$R^4$ $CH_2CH_3$, $COCH_3$ oder $COCH_2OH$

$R^5$ NH2 oder OH

$R^6$ H, OH oder NH2 und

$R^7$ H oder OH

bedeuten, deacyliert.

Im einzelnen geht man dabei wie folgt vor:

Ein Glycosyl-Donor der Formel III wird ausgehend von einem Kohlenhydrat-Vorprodukt, der eine freie Hydroxygruppe am C-1 Atom enthält, und einem Tri-(C1-C4)-Alkylsilylhalogenid in der Gegenwart einer organischen Base wie Pyridin oder Imidazol und eines organischen Lösungsmittels wie Dichlormethan, Dichlorethan bei 20°-60°C hergestellt.

Besonders bevorzugt ist hierbei die Herstellung von Trimethylsilyl- und tert.Butyl(dimethyl)silyl-Derivaten der Formel III.

Zur Glycosylierung eines äq. Anthracyclinons werden in der Regel 1 äq. bis 1.5 äq. Glycosyl-Donor der Formel III benötigt. Als Promotor wird Trifluoromethansulfonsäure tri-(C1-C4)-alkylsilylester, bevorzugt Trifluoromethansulfonsäure trimethylsilylester, Trifluoromethansulfonsäure tert. butyl(dimethyl)silylester oder $BF_3$-Ether eingesetzt. Die Glycosylierung wird in einem wasserfreien organischen Lösungsmittel wie Dichlormethan, Dichlorethan, Ether, Toluol, Acetonitril oder deren Mischungen mit Aceton oder Ethylacetat in der Regel in der Gegenwart eines Trockenmittels wie Molekularsieb oder Bariumsulfat bei -70° C bis 25° C, bevorzugt bei -50° c bis -20° C durchgeführt. Bei der Anwendung von Trifluoro-methansulfonsäureanhydrid als Promotor wird gegebenenfalls eine organische Base wie Triethylamin oder Dimethylaminopyridin zugesetzt.

Die Deacylierung der Glycosid-Produkte wird mittels einer Alkali-Base wie NaOH oder eines Alkoholates wie Natriummethylat durchgeführt.

Die nachfolgenden Beispiele erläutern das erfindungsgemäße Verfahren, ohne es auf die in den Beispielen genannten Verbindungen einzuschränken.

## BEISPIELE

### Beispiel 1

Herstellung von Kohlenhydratbausteinen

Methyl 4-O-p-nitrobenzoyl-2,3,6-tridesoxy-3-trifluoroacetamido-α-L-lyxohexopyranosid (Verbindung 1)
61 g (237 mmol) Methyl 3-N-trifluoroacetyl-daunosaminid wurden in 840 ml Dichlormethan/Pyridin (2:1) gelöst und mit 33 g (177 mmol) p-Nitrobenzoylchlorid versetzt. Der Reaktionsansatz wurde 3 h bei 35° C gerührt, dann in Vakuum eingedampft und mit Toluol nachdestilliert. Der in 600 ml Dichlormethan gelöste Rückstand wurde mit 0.1 N HCl, dann mit Wasser ausgewaschen. Die organische Phase wurde über Natriumsulfat getrocknet und eingedampft. Das Rohprodukt wurde säulenchromatographisch über 600 g Kieselgel (Eluenz: Chloroform/Essigsäureethylester 15:1) gereinigt. Ausbeute: 92 g (95 %); Rf = 0.76 (6:1 $CHCl_3$/EtOAc); Schmp. 65-67° C; $[\alpha]_D$ -183° (c 1.03, chloroform).

4-O-p-Nitrobenzoyl-2,3,6-tridesoxy-3-trifluoroacetamido-L-lyxohexopyranose (Verbindung 2)
25 g (61.5 mmol) Methyl 4-O-p-nitrobenzoyl-3-N-trifluoroacetyl-α-L-daunosaminid wurde in 250 ml 30%iger Trifluoressigsäure suspendiert. Die Reaktionsmischung wurde 10 min unter Rückfluß gehalten. Die abgekühlte Reaktionslösung wurde 2x mit Essigsäureethylester extrahiert. Die organische Phase wurde mit Phosphat-Puffer (0.1 mol $KH_2PO_4$ mit 0.1 mol NaOH auf pH 7.5 eingestellt), dann mit Wasser ausgewaschen und über Natriumsulfat getrocknet. Nach Abdampfen des Filtrates wurde der Rückstand (21 g) aus Ether kristallisiert. Die Mutterlauge (6 g) wurde säulenchromatographisch über 100 g Kieselgel (Elutionsmittel: Chloroform/Essigsäure ethylester 9:1) gereinigt. Ausbeute: 17.4 g (72.4 %); Rf = 0.21 (9:1 $CHCl_3$/EtOAc); Schmp. 213° C; [α] -192° (c 0.86, EtOAc).

3,4-Bis-(trifluoroacetylamino)-2,3,4,6-tetradesoxy-L-lyxohexopyranose (Verbindung 3)
Benzyl 3,4-bis-(trifluoroacetylamino)-2,3,4,6-tetradesoxy-α-L-lyxohexopyranosid (6.5 g) wurde in Eisessig gelöst und in der Gegenwart von 10%igem Pd/C (6.5 g) 48 Stunden hydriert. Dann wurde abfiltriert und das Filtrat in Vacuum eingedampft. Der Rückstand wurde mit 2:1 Methanol/Toluol nachdestilliert. Das resultierende Produkt wurde säulenchromatographisch über Kieselgel (120 g) mit 3:1 Dichlormethan-Methanol gereingt. Ausbeute: 4.39 g; $[\alpha]_D$ -63.3° (c 1, EtOAc).

2,6-Didesoxy-3,4-di-O-p-nitrobenzoyl-L-lyxohexopyranose (Verbindung 4)
2,3-Didesoxy-1,3,4-tri-O-p-nitrobenzoyl-α und ß-L-lyxohexopyranose (2.5 g, 4.2 mmol) wurde in Methanol (60 ml) gelöst und mit aminiertem Kieselgel (Merck, 3 g) versetzt. Die Reaktionsmischung wurde 3 Stunden bei Raumtemperatur gerührt und dann abfiltriert. Nach Abdampfen des Filtrates wurde der Rückstand in Dichlormethan (120 ml) gelöst und mit Phosphat-Puffer (pH 8, 60 ml x 2), dann mit Wasser gewaschen. Die organische Phase wurde über Magnesiumsulfat getrocknet und in Vakuum eingedampft. Der Rückstand wurde säulen chromatographisch über Kieselgel (130 g) mit 7:1 Dichlormethan-Aceton gereinigt. Ausbeute: 1.42 g (76 %).

**Beispiel 2**

Herstellung von 1-O-Trialkylsilyl-Kohlenhydrat-Derivaten

4-O-p-Nitrobenzoyl-2,3,6-tridesoxy-3-trifluoroacetamido-1-O-trimethylsilyl-α-L-lyxohexopyranose (Verbindung 5)
4.6 g (11.7 mmol) 4-O-p-Nitrobenzoyl-2,3,6-tridesoxy-3-trifluoroacetamido-L-lyxohexopyranose wurden in 80 ml Pyridin/Dichlormethan (1:1) gelöst und bei 0° C mit 4.46 ml (35.1 mmol) Trimethylsilylchlorid versetzt. Nach 16 h wurde der Reaktionsansatz mit 100 ml Dichlormethan versetzt und 2x mit je 200 ml Phosphat-Puffer (pH 7.5) ausgewaschen. Die organische Phase wurde über Natriumsulfat getrocknet und in Vakuum eingedampft. Der Rückstand wurde noch mit Toluol nachdestilliert. Das in Hochvakuum getrocknete Rohprodukt wurde säulenchromatographisch über 100 g Kieselgel (Elutionsmittel: Dichlormethan/Petrolether/Aceton 5:5:0.5) gereinigt. Ausbeute: 5.07 g (93.3 %).

1-O-t-Butyl-dimethylsilyl-4-O-p-nitrobenzoyl-2,3,6-tridesoxy-3-trifluoroacetamido-ß-L-lyxohexopyranose (Verbindung 6)
4.05 g (10.3 mmol) 4-O-p-Nitrobenzoyl-3-N-trifluoroacetyl-L-daunosamin wurden in 160 ml Pyridin/1,2-Dichlorethan gelöst und mit 7.7 g (51.5 mmol) t-Butyl-dimethylsilylchlorid versetzt. Nach 16 h Rühren bei 60° C wurde die Reaktionsmischung mit 200 ml Dichlormethan verdünnt und 2x mit Phosphat-Puffer (0.1 mol $KH_2PO_4$ mit 0.1 mol NaOH auf pH 7.5 eingestellt) unter Rückextraktion der Wasserphase ausgewaschen. Die organische Phase wurde über Natriumsulfat getrocknet und in Vakuum eingedampft. Der Rückstand wurde mehrere Male mit Toluol nachdestilliert, bis kein Pyridin vorhanden war, dann säulenchromatographisch über 150 g Kieselgel (Elutionsmittel: Petrolether/Dichlormethan/Aceton 10:10:1) gereinigt. Ausbeute: 4.7 g (90.3 %). Rf = 0.43 (10:10:1 Chloroform/Petrolether/ Aceton); Schmp. 72-74° C; $[\alpha]_D$ -91° (c 1, Chloroform).

3,4-Bis-(trifluoroacetylamino)-1-O-tert.-butyl(dimethyl) silyl-2,3,4,6-tetra-desoxy-L-lyxohexopyranose (Verbindung 7)

Ausgehend von 3,4-Bis-(trifluoroacetylamino)-2,3,4,6-tetradesoxy-L-lyxohexopyranose und tert.-Butyl-(dimethyl)-silylchlorid wurde die Titelverbindung nach der Vorschrift zur Herstellung von Verbindung 6 synthetisiert.

2 6-Didesoxy-3 4-di-O-p-nitrobenzoyl-1-O-tert.-butyl(dimethyl)silyl-L-lyxohexopyranose (Verbindung 8)
Die Titelverbindung wurde ausgehend von 2,6-Didesoxy-3,4-di-O-p-nitrobenzoyl-L-lyxohexopyranose und tert.-Butyl (dimethyl)silyl-chlorid nach der Vorschrift zur Synthese von Verbindung 6 hergestellt.

**Beispiel 3**

Herstellung von 7-O-trimethylsilyl-ϵ-(iso)rhodomycinonen

7-O-Trimethylsilyl-ϵ-rhodomycinon (Verbindung 9)
200 mg (0.429 mmol) ϵ-Rhodomycinon (nach HPLC 92 %) wurden in 10 ml Pyridin/Dichlormethan gelöst und bei 0° C mit 0.180 ml (1.40 mmol) Trimethylsilylchlorid versetzt. Nach 30 min Rühren wurde der Reaktionsansatz mit 20 ml Dichlormethan verdünnt und mit Phosphat-Puffer (pH 7.5) 2x ausgewaschen. Die organische Phase wurde über Natriumsulfat getrocknet, dann eingedampft und der Rückstand wurde mit Toluol zur Entfernung von Pyridinresten nachdestilliert. Das säureempfindliche Produkt wurde säulenchromatographisch über 20 g Kieselgel (Elutionsmittel: Chloroform/Triethylamin 200:1) gereinigt. Ausbeute: 180.6 mg (84 %); $[\alpha]_D$ +279° (c 0.037, Chloroform).

7-O-Trimethylsilyl-ϵ-isorhodomycinon (Verbindung 10)
200 mg (0.45 mmol ϵ-Isorhodomycinon wurden in 10 ml Pyridin/Dichlormethan (1:1) gelöst und bei 0° C mit 0.180 ml (1.398 mmol) Trimethylsilylchlorid versetzt. Nach 30 min Rühren wurde der Reaktionsansatz mit 20 ml Dichlormethan verdünnt und mit Phosphat-Puffer (pH 7.5) 2x ausgewaschen. Die organische Phase wurde über Natriumsulfat getrocknet, dann in Vakuum eingedampft. Der Rückstand wurde mit Toluol zwecks Entfernung von Pyridinresten nachdestilliert. Das säureempfindliche Produkt wurde säulenchromatographisch über 20 g Kieselgel (Elutionsmittel: Chloroform/Triethylamin (200:1)gereinigt. Ausbeute: 180 mg (82 %); [α] +326° (c 0.0061, Chloroform).

**Beispiel 4**

Glycosylierung von Anthracyclinonen mit funktionalisierten Kohlenhydrat-Bausteinen

7-O-(4-O-p-Nitrobenzoyl-2,3,6-tridesoxy-3-trifluoroacetylamino-α-L-lyxohexopyranosyl) -ϵ-isorhodomycinon (Verbindung 11)
ϵ-Isorhodomycinon (6.0 g, 11.2 mmol), Daunosamin-Donor (Verbindung 6, 8.51 g, 16.8 mmol) und Molekularsieb 4 Å (6.0 g) wurden in 5:1 Dichlormethan-Ethylacetat (700 ml) unter Schutzglas suspendiert. Nach der Zugabe von Trifluoromethan-sulfonsäure trimethylsilylester (4.97 g, 22.4 mmol) bei -35° C wurde die Reaktionsmischung 5 Stunden gerührt. Die gekühlte Mischung wurde mit Triethylamin (10 ml) versetzt und abfiltriert. Das Filtrat wurde mit Citrat-Puffer (0.1 molare Citronensäure-Lösung mit 0.1 molarer NaOH auf pH5 eingestellt) dann mit Eiswasser zweimal gewaschen. Die organische Phase wurde über Natriumsulfat getrocknet und in Vakuum eingedampft. Der Rückstand (12.6 g) wurde säulenchromatographisch über Kieselgel (450 g) mit 200:10:1 Chloroform-Ethylacetat-Ameisensäure gereinigt. Ausbeute: 7.15 g (78 %); Rf = 0.33 (Lösungsmittel - siehe oben)

7-O-(4-O-p-Nitrobenzoyl-2,3,6-tridesoxy-3-trifluoroacetylamino-α-L-lyxohexopyranosyl)-ϵ-rhodomycinon (Verbindung 12)
Die Titelverbindung 12 wurde ausgehend von ϵ-Rhodomycinon und Daunosamin-Donor (Verbindung 6) nach der Vorschrift zur Herstellung von Verbindung 11 synthetisiert.

7-O-(4-O-p-Nitrobenzoyl-2,3,6-tridesoxy-3-trifluoroacetylamino-α-L-lyxohexopyranosyl)-daunomycinon (Verbindung 13)
Die Titelverbindung 13 wurde ausgehend von Daunomycinon und Daunosamin-Donor (Verbindung 6) nach der Vorschrift zur Synthese von Verbindung 11 hergestellt.

7-O-(3,4-Bis-(trifluouroacetylamino)-2,3,4,6-tetra-desoxy-α-L-lyxohexopyranosyl)-4-O-methyl-10-O-p-nitrobenzoyl-ß-rhodomycinon (Verbindung 14)
4-O-Methyl-10-O-p-nitrobenzoyl-ß-rhodomycinon (2.64 g, 4.80 mmol), 3,4-Bis-trifluoroacetylamino-Zucker (Verbindung 7, 2.17 g, 4.80 mmol) und Molekularsieb 4 Å (3.0 g) wurden in Dichlormethan (220 ml) gelöst und bei -35° C mit Trimethylsilyltriflat (1.2ml) versetzt. Die Reaktionsmischung wurde 3 Stunden bei -35° C gerührt, dann mit Triethylamin (2.5 ml) versetzt und abfiltriert. Das Filtrat wurde mit Phosphat-Puffer (pH 7, 60 ml x 2), dann mit Wasser gewaschen, getrocknet (Natruimsulfat) und in Vakuum eingedampft. Der

Rückstand wurde säulenchromatographisch über Kieselgel (220 g) mit 15:1 Chloroform-Aceton gereinigt. Ausbeute: 3.38 g (81 %), Rf = 0.37 (15:1 Chloroform-Aceton); Schmp. 222° C; alpha D + 250° (c 0.05, Chloroform)

7-O-(3,4-Bis-O-p-nitrobenzoyl-2,6-didesoxy-alpha-L-lyxo-hexopyranosyl)-4-O-methyl-10-O-p-nitrobenzoyl-ß-rhodomycinon (Verbindung 15)

Die Titelverbindung wurde ausgehend von 2-Desoxy-fucose-Donor (Verbindung 8) und 4-O-Methyl-10-O-p-nitrobenzoyl-ß-rhodomycinon nach der Vorschrift zur Herstellung von Verbindung 14 synthetisiert.

**Beispiel 5**

Entblockierung von Anthracyclinen

Die in Beispiel 4 beschriebenen Anthracyclin-Derivate wurden nach bekanntem Verfahren, wie in der folgenden allgemeine Vorschrift beschrieben, entblockiert:

Geschützte Anthracyclin-Verbindung wurde in Methanol oder Chloroform-Methanol gelöst und bei 0° C mit 1 N NaOH versetzt. Nach dem Ablaufen der Reaktion wurde die Reaktionsmischung mit 1 N HCl neutralisiert. Nach der üblichen Aufarbeitung wurde die entblockierte Verbindung über RP-18 Kieselgel oder Aminokieselgel gereinigt.

Nach dieser Vorschrift wurden folgende Verbindungen hergestellt:

7-O-(3-Amino-2,3,6,tridesoxy-alpha-L-lyxohexopyranosyl)-ε-isorhodomycinon (Verbindung16)

7-O-(3-Amino-2,3,6-tridesoxy-alpha-L-lynxohexopyranosyl)-ε-rhodomycinon (Verbindung 17)

7-O-(3-Amino-2,3,6-tridesoxy-α-L-lyxohexopyranosyl)-daunomycinon (Verbindung 18)

7-O-(3,4-Diamino-2,3,4,6-tetra-desoxy-α-L-lyxohexopyranosyl)-4-O-methyl-ß-rhodomycinon (Verbindung 19)

7-O-(2,6-Didesoxy-α-L-lyxohexopyranosyl)-4-O-methyl-ß-rhodomycinon (Verbindung 20)

**Patentansprüche**

**1.** Verfahren zur Herstellung eines Anthracyclins der Formel I,

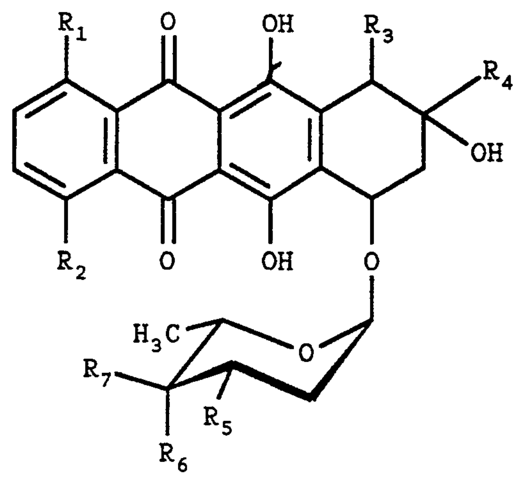

I

worin

$R^1$ H oder OH

$R^2$ H, OH oder $OCH_3$

$R^3$ H, $COOCH_3$, OH oder O-Acyl-Schutzgruppe

$R^4$ $CH_2CH_3$, $COCH_3$, $COCH_2OH$ oder $COCH_2O$-Acyl-Schutzgruppe

$R^5$ $NH_2$, NH-Acyl-Schutzgruppe, OH oder O-Acyl-Schutzgruppe

$R^6$ H, OH, O-Acyl-Schutzgruppe, $NH_2$ oder NH-Acyl-Schutzgruppe

$R^7$ H, OH oder O-Acyl-Schutzgruppe

bedeuten, dadurch gekennzeichnet, daß man eine Anthracyclinon-Verbindung der Formel II,

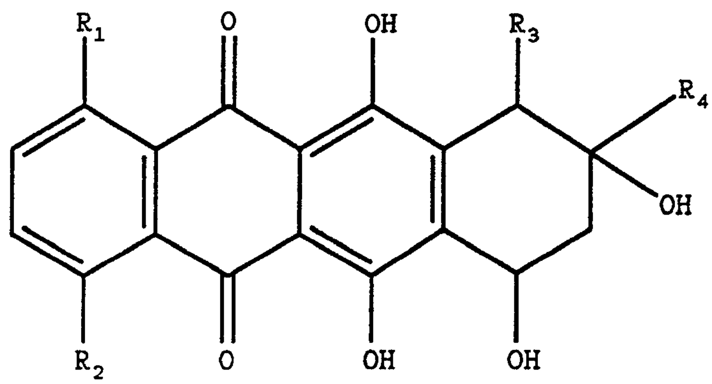

II

worin

$R^1$ H oder OH

$R^2$ H, OH oder $OCH_3$

$R^3$ H, $COOCH_3$ oder O-Acyl-Schutzgruppe und

$R^4$ $CH_2CH_3$, $COCH_3$, $COCH_2$-O-Acyl-Schutzgruppe

bedeuten,

mit einem funktionalisierten Kohlenhydrat-Baustein der Formel III,

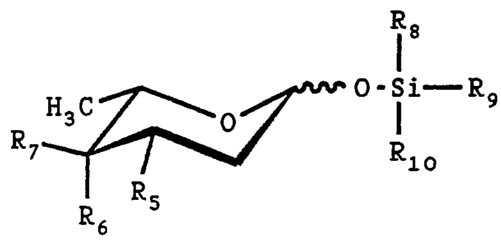

III

worin

$R^5$ NH-Acyl-Schutzgruppe oder O-Acyl-Schutzgruppe

$R^6$ H, NH-Acyl-Schutzgruppe oder O-Acyl-Schutzgruppe

$R^7$ H oder O-Acyl-Schutzgruppe und

$R^8$, $R^9$ und $R^{10}$ $(C_1\text{-}C_4)$-Alkyl

bedeuten,

in Gegenwart eines Promotors in einem organischen wasserfreien Lösungsmittel, gegebenenfalls in Präsenz einer Base oder eines Säurefängers und eines Trockenmittels bei -50° C bis 25° C zu einer Verbindung der Formel I, worin die Reste $R^1$, $R^2$, $R^3$, $R^4$, $R^5$, $R^6$ und $R^7$ die oben bei den Formeln II und III definierte Bedeutung beibehalten und diese Verbindungen gegebenenfalls zu zytostatisch wirksamen Verbindungen der Formel I,

worin

$R^1$ H oder OH

$R^2$ H, OH oder $OCH_3$

$R^3$ H, $COOCH_3$ oder OH

$R^4$ $CH_2CH_3$, $COCH_3$ oder $COCH_2OH$

$R^5$ $NH_2$ oder OH

$R^6$ H, OH oder $NH_2$ und

$R^7$ H oder OH

bedeuten, deacyliert.

2. Verfahren nach Anspruch 1, dadurch gekennzeichnet, daß eine Verbindung der Formel I,

worin

$R^1$ H oder OH

$R^2$ H, OH oder $OCH_3$

$R^3$ H, $COOCH_3$ oder O-Acyl-Schutzgruppe

$R^4$ $CH_2CH_3$, $COCH_3$ oder $COCH_2O$-Acyl-Schutzgruppe
$R^5$ NH-Acyl-Schutzgruppe oder O-Acyl-Schutzgruppe
$R^6$ H, O-Acyl-Schutzgruppe oder NH-Acyl-Schutzgruppe
$R^7$ H oder O-Acyl-Schutzgruppe
bedeuten, hergestellt wird.

3. Verfahren nach mindestens einem der Ansprüche 1 und 2, dadurch gekennzeichnet, daß eine Acyl-Schutzgruppe eine Acetyl-, Mono-, Di- oder Trihalogenacetylgruppe mit Halogen, Fluor oder Chlor oder eine Benzoyl- oder p-Nitrobenzoylgruppe darstellt.

4. Verfahren nach mindestens einem der Ansprüche 1 und 2, dadurch gekennzeichnet, daß als Acyl-Schutzgruppe für Aminogruppen eine Trifluoroacetylgruppe und für Hydroxygruppen eine Acetyl-, Chloroacetyl-, Trifluoroacetyl-, Benzoyl- oder p-Nitrobenzoyl-Gruppe verwendet wird.

5. Verfahren nach Anspruch 1, dadurch gekennzeichnet, daß als Promotor Trifluoromethansulfonsäure tri-$(C_1$-$C_4)$-alkylsilylester oder -anhydrid oder $BF_3$-Ether verwendet wird.

6. Verfahren nach Anspruch 1, dadurch gekennzeichnet, daß als Promotor Trifluoromethansulfansäure-trimethylsilyl oder tert. Butyl(dimethyl)silyl-ester verwendet wird.

7. Verfahren nach Anspruch 1, dadurch gekennzeichnet, daß als organisches Lösungsmittel Dichloromethan, Dichloroethan, Ether, Toluol, Acetonitril oder eine ihrer Mischungen mit Aceton oder Ethyl-acetat verwendet wird.

8. Verfahren nach Anspruch 1, dadurch gekennzeichnet, daß mittels einer Alkalilauge oder eines Alkoholates deacyliert wird.

9. Funktionalisierte Kohlenhydrat-Bausteine der Formel III, worin
$R^5$ NH-Acyl-Schutzgruppe oder O-Acyl-Schutzgruppe
$R^6$ H, NH-Acyl-Schutzgruppe oder O-Acyl-Schutzgruppe
$R^7$ H oder O-Acyl-Schutzgruppe und
$R^8$, $R^9$ und $R^{10}$ $(C_1$-$C_4)$-Alkyl
bedeuten, wobei eine Acyl-Schutzgruppe für Aminogruppen Trifluoroacetyl und für Hydroxygruppen Acetyl, Chloroacetyl, Trifluoroacetyl, Benzoyl oder p-Nitrobenzoyl ist.

**Patentansprüche für folgende Vertragsstaaten:ES,GR**

1. Verfahren zur Herstellung eines Anthracyclins der Formel I,

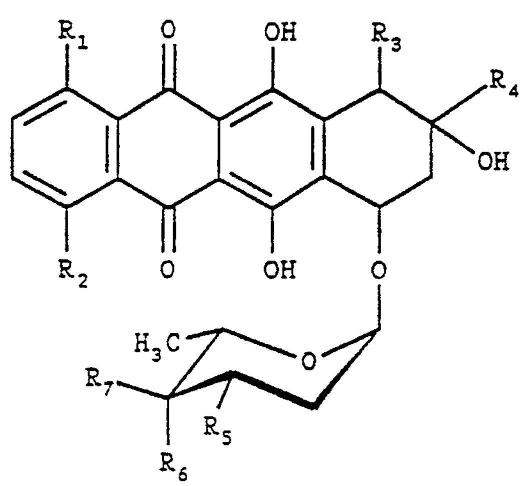

I

worin
$R^1$ H oder OH

$R^2$ H, OH oder $OCH_3$
$R^3$ H, $COOCH_3$, OH oder O-Acyl-Schutzgruppe
$R^4$ $CH_2CH_3$, $COCH_3$, $COCH_2OH$ oder $COCH_2O$-Acyl-Schutzgruppe
$R^5$ $NH_2$, NH-Acyl-Schutzgruppe, OH oder O-Acyl-Schutzgruppe
$R^6$ H, OH, O-Acyl-Schutzgruppe, $NH_2$ oder NH-Acyl-Schutzgruppe
$R^7$ H, OH oder O-Acyl-Schutzgruppe
bedeuten, dadurch gekennzeichnet, daß man eine Anthracyclinon-Verbindung der Formel II,

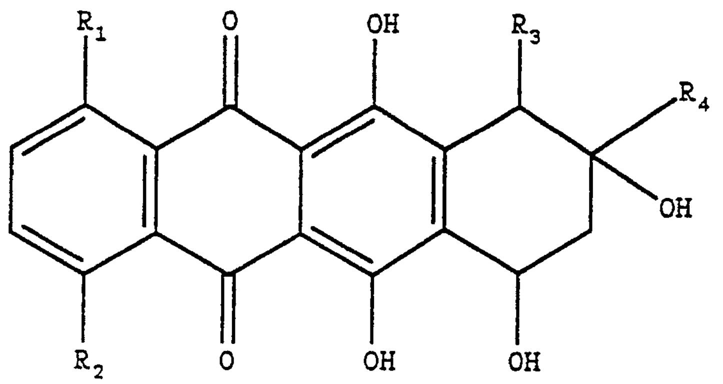

II

worin
$R^1$ H oder OH
$R^2$ H, OH oder $OCH_3$
$R^3$ H, $COOCH_3$ oder O-Acyl-Schutzgruppe und
$R^4$ $CH_2CH_3$, $COCH_3$, $COCH_2$-O-Acyl-Schutzgruppe
bedeuten,
mit einem funktionalisierten Kohlenhydrat-Baustein der Formel III,

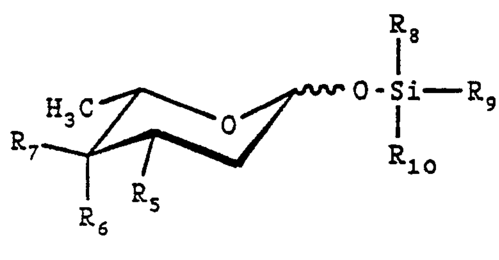

III

worin
$R^5$ NH-Acyl-Schutzgruppe oder O-Acyl-Schutzgruppe
$R^6$ H, NH-Acyl-Schutzgruppe oder O-Acyl-Schutzgruppe
$R^7$ H oder O-Acyl-Schutzgruppe und
$R^8$, $R^9$ und $R^{10}$ ($C_1$-$C_4$)-Alkyl
bedeuten,
in Gegenwart eines Promotors in einem organischen wasserfreien Lösungsmittel, gegebenenfalls in Präsenz einer Base oder eines Säurefängers und eines Trockenmittels bei -50° C bis 25° C zu einer Verbindung der Formel I, worin die Reste $R^1$, $R^2$, $R^3$, $R^4$, $R^5$, $R^6$ und $R^7$ die oben bei den Formeln II und III definierte Bedeutung beibehalten und diese Verbindungen gegebenenfalls zu zytostatisch wirksamen Verbindungen der Formel I, worin
$R^1$ H oder OH
$R^2$ H, OH oder $OCH_3$
$R^3$ H, $COOCH_3$ oder OH
$R^4$ $CH_2CH_3$, $COCH_3$ oder $COCH_2OH$
$R^5$ NH2 oder OH
$R^6$ H, OH oder $NH_2$ und

$R^7$ H oder OH
bedeuten, deacyliert.

2. Verfahren nach Anspruch 1, dadurch gekennzeichnet, daß eine Verbindung der Formel I, worin
   $R^1$ H oder OH
   $R^2$ H, OH oder $OCH_3$
   $R^3$ H, $COOCH_3$ oder O-Acyl-Schutzgruppe
   $R^4$ $CH_2CH_3$, $COCH_3$ oder $COCH_2O$-Acyl-Schutzgruppe
   $R^5$ NH-Acyl-Schutzgruppe oder O-Acyl-Schutzgruppe
   $R^6$ H, O-Acyl-Schutzgruppe cder NH-Acyl-Schutzgruppe
   $R^7$ H oder O-Acyl-Schutzgruppe
   bedeuten, hergestellt wird.

3. Verfahren nach mindestens einem der Ansprüche 1 und 2, dadurch gekennzeichnet, daß eine Acyl-Schutzgruppe eine Acetyl-, Mono-, Di- oder Trihalogenacetylgruppe mit Halogen, Fluor oder Chlor oder eine Benzoyl- oder p-Nitrobenzoylgruppe darstellt.

4. Verfahren nach mindestens einem der Ansprüche 1 und 2, dadurch gekennzeichnet, daß als Acyl-Schutzgruppe für Aminogruppen eine Trifluoroacetylgruppe und für Hydroxygruppen eine Acetyl-, Chloroacetyl-, Trifluoroacetyl-, Benzoyl- oder p-Nitrobenzoyl-Gruppe verwendet wird.

5. Verfahren nach Anspruch 1, dadurch gekennzeichnet, daß als Promotor Trifluoromethansulfonsäure tri-($C_1$-$C_4$)-alkylsilylester oder -anhydrid oder $BF_3$-Ether verwendet wird.

6. Verfahren nach Anspruch 1, dadurch gekennzeichnet, daß als Promotor Trifluoromethansulfansäure-trimethylsilyl oder tert. Butyl(dimethyl)silyl-ester verwendet wird.

7. Verfahren nach Anspruch 1, dadurch gekennzeichnet, daß als organisches Lösungsmittel Dichloromethan, Dichloroethan, Ether, Toluol, Acetonitril oder eine ihrer Mischungen mit Aceton oder Ethyl-acetat verwendet wird.

8. Verfahren nach Anspruch 1, dadurch gekennzeichnet, daß mittels einer Alkalilauge oder eines Alkoholates deacyliert wird.